# EUROPEAN PATENT SPECIFICATION

(11) **EP 4 331 484 B1**
(45) Date of publication and mention of the grant of the patent: **05.08.2026**
(21) Application number: 23193444.9
(22) Date of filing: 25.08.2023
(51) Int. Cl.: A61B 5/11, A61B 5/16, A61B 5/00, A61B 5/024, G16H 50/20, G16H 50/30

(54) **MENTAL/PHYSICAL STATE EVALUATION SYSTEM AND MENTAL/PHYSICAL STATE EVALUATION METHOD**
SYSTEM ZUR BEWERTUNG DES MENTALEN/PHYSIKALISCHEN ZUSTANDS UND VERFAHREN ZUR BEWERTUNG DES MENTALEN/PHYSIKALISCHEN ZUSTANDS
SYSTÈME D'ÉVALUATION D'ÉTAT MENTAL/PHYSIQUE ET PROCÉDÉ D'ÉVALUATION D'ÉTAT MENTAL/PHYSIQUE

(30) Priority: 31.08.2022 JP 2022137591; 05.07.2023 JP 2023110567
(43) Date of publication of application: 06.03.2024
(73) Proprietor: ASICS Corporation, Kobe-shi Hyogo 650-8555 (JP)
(72) Inventor: Abe, Satoru, 650-8555, Hyogo (JP); Takashima, Shingo, Hyogo, 650-8555 (JP)
(74) Representative: Marks & Clerk LLP

(56) References cited:
- EP-A1- 3 636 156
- WO-A1-2021/068038
- WO-A1-2021/207852
- WO-A1-2022/137498
- US-A1- 2015 208 975
- US-A1- 2020 061 378

## Description

### BACKGROUND

### 1. Technical Field

The present disclosure relates to a mental/physical state evaluation system. In particular, the present disclosure relates to a technique for evaluating a relationship between a body motion and a mental state.

### 2.Description of the Related Art

Generally, various factors are involved in determining whether an athlete performs well or bad in sports. In particular, when an athlete falls into a slump or is injured, it is considered that this is affected by the stability of the mental state of the athlete in many cases. Here, a technique for quantitatively evaluating a user's emotion is known (see, for example, JP 2021-110781 A).

WO 2022137498 A1 describes a system where parameters such as "landing impact", "running efficiency", "stability", and "range of motion", are displayed together with a subjective parameter value manually input by the user to indicate a feeling of fast running, fatigue, or pain.

Even if the athlete recognizes that the cause of the bad condition of the athlete is in the mental state, it is not easy to find an expert who the athlete can consult with, and even if the athlete can receive a counseling, whether the counseling is effective cannot be known until the athlete receives the counseling. It is predicted that it takes a relatively long time to determine the effect of the counseling. Therefore, it is not easy to easily improve such a mental state.

### SUMMARY

The present disclosure has been made in view of such a situation, and an object thereof is to provide a technique that enables easily grasping and improving the relationship between the mental state and the exercise performance by oneself.

In order to solve the above-described problem, a system and method according to the independent claims are provided. Preferred embodiments are described in the dependent claims.

Furthermore a mental/physical state evaluation system according to one aspect of the present disclosure includes a reproducibility evaluator configured to determine a reproducibility evaluation value on a basis of an analysis result regarding reproducibility of a predetermined body motion of a subject; a mental evaluator configured to determine a mental evaluation value on a basis of an analysis result regarding a mental state of the subject; an evaluation value storage configured to store the reproducibility evaluation value and the mental evaluation value in association with each other; and a result outputter configured to output information indicating relevance between the mental evaluation value and quality of the reproducibility evaluation value.

Another aspect of the present disclosure is a mental/physical state evaluation method. This method includes determining a reproducibility evaluation value on a basis of an analysis result regarding reproducibility of a predetermined body motion of a subject; determining a mental evaluation value on a basis of an analysis result regarding a mental state of the subject; storing the reproducibility evaluation value and the mental evaluation value in association with each other; and outputting information indicating relevance between the mental evaluation value and quality of the reproducibility evaluation value.

Note that arbitrary combinations of the above components and modifications of the expressions of the present disclosure among methods, apparatuses, systems, computer programs, data structures, recording media, and the like are also valid as aspects of the present disclosure.

### BRIEF DESCRIPTION OF THE DRAWINGS

Fig. 1 is a diagram illustrating a schematic configuration of a mental/physical state evaluation system that acquires and analyzes information indicating a motion state of a subject and information indicating a mental state of the subject;
Fig. 2 is a functional block diagram illustrating each element of the mental/physical state evaluation system;
Fig. 3 is a diagram illustrating a process of estimating a position of an anatomical feature point from an image obtained by photographing a subject putting in golf as an example;
Fig. 4 is a diagram illustrating an example in which the positions of a plurality of feature points in a case where the same putting motion is repeated three times are indicated in two-dimensional coordinates;
Fig. 5 is a graph illustrating a time history characteristic of a motion feature value in a case where the same putting motion is repeated three times;
Fig. 6 is a diagram illustrating a screen example of comparing and displaying operation reproducibility in a case where the mental state is good and operation reproducibility in a case where the mental state is bad;
Fig. 7 is a diagram illustrating a process of estimating a position of an anatomical feature point from an image obtained by photographing a running subject; and
Fig. 8 is a diagram illustrating a process of estimating a position of an anatomical feature point from an image obtained by photographing a subject performing a trial of a trick.

### DETAILED DESCRIPTION

The present disclosure will now be described by reference to the preferred embodiments. This does not intend to limit the scope of the present invention, but to exemplify the invention.

Hereinafter, a mental/physical state evaluation system and a mental/physical state evaluation program will be exemplarily described with reference to the drawings on the basis of preferred embodiments of the present disclosure. In some embodiments and the modification examples, the same or equivalent components are denoted by the same reference signs, and redundant description will be omitted as appropriate.

### First Embodiment

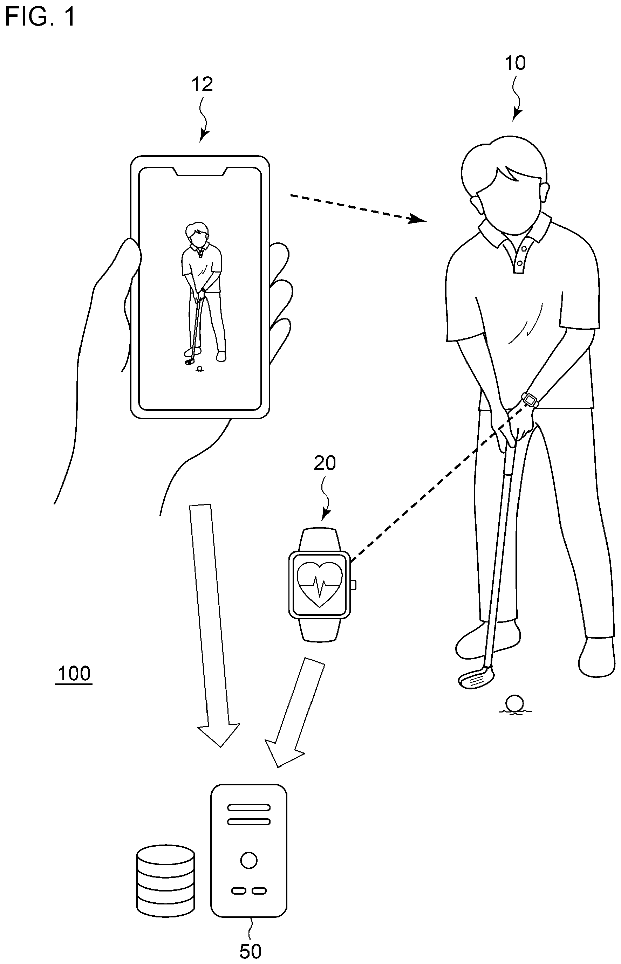

Fig. 1 illustrates a schematic configuration of a mental/physical state evaluation system that acquires and analyzes information indicating a motion state of a subject and information indicating a mental state of the subject; In the example of the present embodiment, a subject 10 putting in golf is photographed by a camera function of an information terminal 12, and the photographed image is transmitted to a mental/physical state evaluation server 50 as information indicating the motion state. The subject 10 wears, for example, on a wrist thereof, a measurement device 20 such as a smart watch that acquires biological information, for example, information indicating a mental state such as a heart rate, a blood oxygen level, and a pupil response, and information indicating a motion state of the arm movement and the like. Information indicating the mental state of the subject 10 and information indicating the motion state of the subject 10 are transmitted from the measurement device 20 to the mental/physical state evaluation server 50 via a predetermined communication path. For example, the measurement device 20 may be connected to a predetermined program of the information terminal 12 by short-distance wireless communication to synchronize the information, and the information may be transmitted from the information terminal 12 to the mental/physical state evaluation server 50.

The mental/physical state evaluation server 50 is a server on a network that quantifies and evaluates the reproducibility of the motion of the subject 10 and quantifies and evaluates the mental state of the subject 10, on the basis of the information indicating the motion state and the information indicating the mental state. The information terminal 12 and the mental/physical state evaluation server 50 are connected via a network such as wireless communication or the Internet. The mental/physical state evaluation server 50 receives information from a plurality of subjects 10, determines an evaluation value for each subject 10, and manages the information. The mental/physical state evaluation server 50 stores a reproducibility evaluation value and a mental evaluation value in association with each other, and outputs information indicating the relevance between mental evaluation value and the quality of the reproducibility evaluation value to the information terminal 12. The subject 10 can grasp the relevance between the reproducibility of the motion and the mental state by viewing the result displayed on the information terminal 12. The relevance between the quality of the mental state and the quality of the motion reproducibility is visualized, and the reproducibility evaluation values corresponding to a plurality of situations having different mental states are compared and displayed. Therefore, for example, in a case where the exercise performance has deteriorated, the subject 10 can find a clue to improvement in the mental state by oneself.

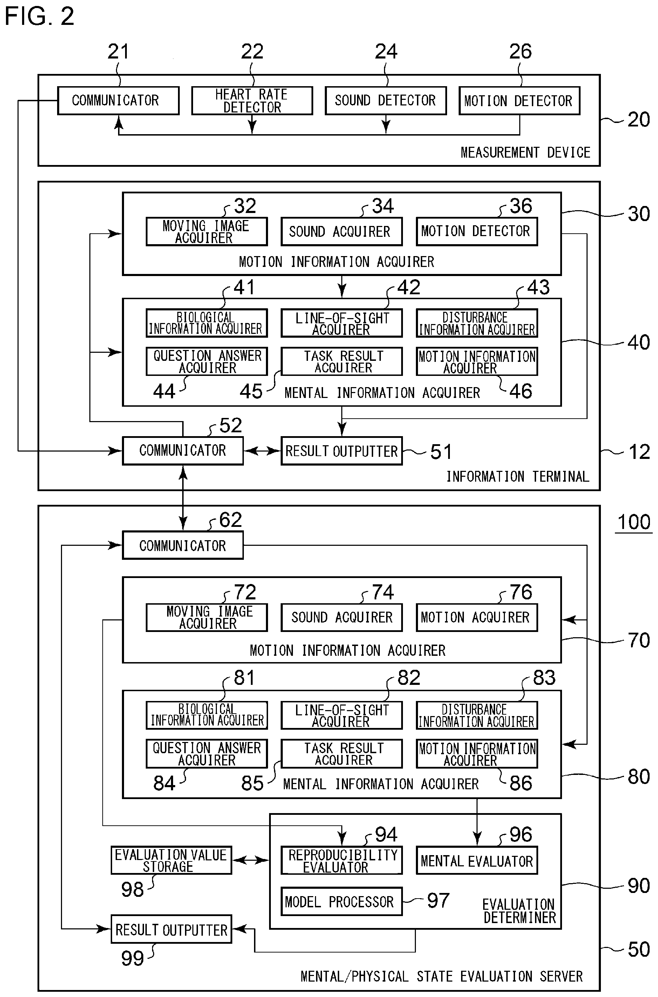

Fig. 2 is a functional block diagram illustrating each element of the mental/physical state evaluation system 100. This drawing depicts a block diagram focusing on functions, and these functional blocks can be implemented in various forms by hardware, software, or a combination thereof. The information terminal 12 and the mental/physical state evaluation server 50 may be constituted by a mobile terminal or a computer mainly including a central processing unit (CPU), a graphics processing unit (GPU), a random access memory (RAM), a read only memory (ROM), an auxiliary storage device, a display device, a communication device, a camera module, and the like, and a program stored in the mobile terminal or the computer.

The mental/physical state evaluation system 100 according to the present embodiment includes the measurement device 20, the information terminal 12, and the mental/physical state evaluation server 50. However, it is conceivable that the mental/physical state evaluation system 100 is realized by various hardware elements and software elements. For example, it is not essential to include the measurement device 20 as an element of the mental/physical state evaluation system 100, and various general-purpose devices can be used as the measurement device 20, or the information terminal 12 can be used as the measurement device 20 by utilizing various functions of the information terminal 12. On the premise that the motion state or the mental state of the subject is measured using a general-purpose device or a function of the information terminal 12 as the measurement device 20, the mental/physical state evaluation system 100 may be constituted only by the information terminal 12 and the mental/physical state evaluation server 50.

Alternatively, by causing the information terminal 12 to have all the functions of the information terminal 12 and the characteristic functions of the mental/physical state evaluation server 50, the information terminal may be constituted by a combination of the measurement device 20 and the information terminal 12, or may be constituted substantially only by the information terminal 12. Alternatively, the mental/physical state evaluation system 100 may be constituted substantially only by the mental/physical state evaluation server 50 by causing the mental/physical state evaluation server 50 to have all the characteristic functions of the present embodiment and using a general-purpose device as the information terminal 12 and the measurement device 20. Regardless of the aspect of the hardware configuration, it is sufficient if the mental/physical state evaluation system 100 includes at least software elements included in the information terminal 12 and the mental/physical state evaluation server 50 in the drawings.

The measurement device 20 includes a communicator 21, a heart rate detector 22, a sound detector 24, and a motion detector 26. The measurement device 20 is, for example, a wearable device such as a smart watch worn on the wrist of the subject or smart glasses that are worn on the eyes like glasses or sunglasses. The heart rate detector 22 is a function of detecting the heart rate of the subject wearing the measurement device 20, and includes, for example, an optical heart rate meter as hardware. The sound detector 24 is a function of inputting the sound of the surroundings, and includes a sound processing device including, for example, a microphone as hardware. The motion detector 26 is a function of detecting the motion of the subject wearing the measurement device 20, and includes, as hardware, for example, a motion sensor such as a 9-axis sensor that detects the motion of the arm, a camera module that detects the line of sight of the subject 10, a pressure sensor that detects the foot pressure or the grip strength of the subject 10, or the like.

The information terminal 12 includes a motion information acquirer 30, a mental information acquirer 40, a result outputter 51, and a communicator 52. The information terminal 12 is a mobile terminal such as a smartphone or a tablet terminal capable of capturing a moving image, displaying an image, audio recording, audio input/output, detecting an operation, and transmitting/receiving information.

The motion information acquirer 30 acquires information indicating a motion state of the subject 10 who repeatedly performs a predetermined body motion. The motion information acquirer 30 includes a moving image acquirer 32, a sound acquirer 34, and a motion detector 36.

The moving image acquirer 32 is a function of acquiring an image obtained by photographing the subject 10 repeatedly performing a predetermined body motion, and includes, for example, a camera module as hardware. For example, the moving image acquirer 32 acquires a video image obtained by capturing the subject 10 repeatedly performing a putting motion and a swing motion in golf a plurality of times as illustrated in Fig. 1.

The sound acquirer 34 is a function of inputting the sound of the surroundings, and includes a sound processing device including, for example, a microphone as hardware. The sound acquirer 34 may acquire sound information input by the measurement device 20 via the communicator 52. For example, as illustrated in Fig. 1, the sound acquirer 34 acquires a ball impact sound, a swing sound, and the like when the subject 10 putts or swings in golf.

The motion detector 36 is a function of detecting the motion of the subject carrying the information terminal 12, and includes, for example, a motion sensor such as a 9-axis sensor as hardware. The motion detector 36 may acquire motion information detected by the measurement device 20 via the communicator 52. In a case where the measurement device 20 is smart glasses, information of the movement of the line of sight of the subject 10 detected by the measurement device 20 may be acquired as the motion information.

The mental information acquirer 40 acquires information indicating the mental state of the subject 10. The mental information acquirer 40 further acquires disturbance information indicating the degree of disturbance stimulus that can affect the mental state of the subject 10. The timing at which the mental information acquirer 40 acquires the information indicating the mental state of the subject 10 may be at the time of performing a predetermined body motion.

The mental information acquirer 40 includes a biological information acquirer 41, a line-of-sight acquirer 42, a disturbance information acquirer 43, a question answer acquirer 44, a task result acquirer 45, and an action information acquirer 46. The biological information acquirer 41 acquires information indicating a mental state including biological information such as a heart rate and a blood oxygen level from the measurement device 20 via the communicator 52. The line-of-sight acquirer 42 detects movement of the line of sight of the subject from a moving image acquired by the motion information acquirer 30. Alternatively, the line-of-sight acquirer 42 may acquire information of the movement of the line of sight of the subject detected by the measurement device 20 such as smart glasses.

The disturbance information acquirer 43 acquires disturbance information indicating a degree of disturbance stimulus that can affect the mental state when the subject 10 performs a predetermined body motion. The disturbance information is input by a touch panel operation by the subject 10. The disturbance stimulus is, for example, an auditory stimulus such as noise, a visual stimulus that induces attention, or the like. The disturbance stimulus may be information indicating an external environment at the time when the subject 10 performs a predetermined body motion of the subject 10, or may be a stimulus as an external influence intentionally given to measure an influence on the mental state. The information indicating the external environment may be, for example, an objective value such as weather, temperature, humidity, date, time, day of the week, season, type of turf, latitude, longitude, or altitude. The disturbance stimulus to the concentration of the subject is, for example, a stimulus that disturbs the subject's consciousness, such as a memory task or a calculation task. The disturbance stimulus to the attention of the subject is, for example, a stimulus that disturbs the subject's sense such as vision, hearing, or touch. The disturbance information may be, for example, information obtained by the subject 10 selecting and inputting an item of external stimulus and the subject 10 subjectively determining the degree of stimulus and inputting a numerical value.

The question answer acquirer 44 acquires an answer to a question related to the mental information through an input by a touch panel operation of the subject 10. The question related to mental information is, for example, a question item of a questionnaire for estimating the mental state. Examples of questions include "Q. I'm nervous.", "Q. I'm focused.", and "Q. I feel good." as question items for measuring subjective mental state. Examples of answers to these questions include answers on a five-grade scale of "1. I don't think so at all.", "2. I don't think so.", "3. I'm not sure.", "4. I think so.", and "5. I think so very much.".

The task result acquirer 45 acquires a result of a cognitive task for measuring concentration or attention through input by a touch panel operation by the subject 10. The result of the cognitive task for measuring concentration or attention is, for example, a score such as a correct answer rate or a response time calculated on the basis of an input by the subject 10 in a test for measuring concentration or ease of drawing attention. Examples of the cognitive task include a test in which a screen in which a large number of characters indicating names of colors such as "Yellow", "Blue", "Green", and "Red" are displayed in a state in which characters displayed in colors indicated by the characters and characters intentionally displayed in colors different from the colors indicated by the characters are arranged in a mixed manner is displayed and the subject 10 is caused to select ones in which the characters and the display colors match. The subject 10 selects ones in which the characters and the display colors match, and the correct answer rate and the time at which all correct answers are input are detected as the test result. In this manner, the concentration and attention of the subject 10 can be measured by the test that is likely to be misread at a glance, and the mental state at that time can be estimated.

The action information acquirer 46 acquires information regarding an action or an effort performed by the subject 10 to improve or maintain the body motion or the mental state through an input by a touch panel operation by the subject 10. The action information is, for example, an item name or a numerical value indicating a type of action such as a practice amount or mindfulness.

The result outputter 51 displays information indicating the motion state acquired by the motion information acquirer 30 and information indicating the mental state acquired by the mental information acquirer 40 on the screen and transmits the information to the mental/physical state evaluation server 50 via the communicator 52. In addition, the result outputter 51 acquires various evaluation values determined by the mental/physical state evaluation server 50 via the communicator 52, and displays the evaluation values on the screen. The result outputter 51 may have a function of posting information indicating the motion state and information indicating the mental state such as an acquired moving image, and information such as an evaluation value acquired from the mental/physical state evaluation server 50 to various social networking services (SNSs) and disclosing the information within a specific range, or collecting information from the SNSs and displaying the collected information.

The mental/physical state evaluation server 50 includes a communicator 62, a motion information acquirer 70, a mental information acquirer 80, an evaluation determiner 90, an evaluation value storage 98, and a result outputter 99.

The motion information acquirer 70 is a function corresponding to the motion information acquirer 30 of the information terminal 12, and includes a moving image acquirer 72, a sound acquirer 74, and a motion acquirer 76. The moving image acquirer 72 acquires an image obtained by photographing the subject 10 repeatedly performing a predetermined body motion from the information terminal 12 via the communicator 62. The sound acquirer 74 acquires sound information from the information terminal 12 via the communicator 62. The motion acquirer 76 acquires information indicating motion such as a detection result of a motion sensor or a detection result of the line of sight from the information terminal 12 via the communicator 62.

The mental information acquirer 80 is a function corresponding to the mental information acquirer 40 of the information terminal 12, and includes a biological information acquirer 81, a line-of-sight acquirer 82, a disturbance information acquirer 83, a question answer acquirer 84, a task result acquirer 85, and an action information acquirer 86. The biological information acquirer 81 acquires information indicating the mental state including biological information such as a heart rate and a blood oxygen level of the subject detected by the measurement device 20 such as a smart watch from the information terminal 12 via the communicator 62. The line-of-sight acquirer 82 detects movement of the line of sight of the subject from a moving image acquired from the information terminal 12 via the communicator 62. Alternatively, the line-of-sight acquirer 82 may acquire information of the movement of the line of sight of the subject detected by the measurement device 20 such as smart glasses from the information terminal 12 via the communicator 62. Note that, in the present embodiment, the motion information acquirer 30 and the mental information acquirer 40 in the information terminal 12 and the motion information acquirer 70 and the mental information acquirer 80 in the mental/physical state evaluation server 50 are described as similar functions. However, the motion information acquirer and the mental information acquirer may be configured to be provided only on one of the information terminal 12 side and the mental/physical state evaluation server 50 side, or may be configured to be provided with functions distributed to both sides.

The disturbance information acquirer 83 acquires, from the information terminal 12 via the communicator 62, disturbance information indicating a degree of disturbance stimulus that can affect the mental state when the subject 10 performs a predetermined body motion. The question answer acquirer 84 acquires an answer to a question related to the mental information from the information terminal 12 via the communicator 62. The task result acquirer 85 acquires a result of a cognitive task for measuring concentration or attention from the information terminal 12 via the communicator 62. The action information acquirer 86 acquires information regarding an action or an effort performed by the subject 10 to improve or maintain the body motion or the mental state from the information terminal 12 via the communicator 62.

The evaluation determiner 90 analyzes and quantifies the information indicating the motion state and the information indicating the mental state, determines the evaluation value, and stores the evaluation value in the evaluation value storage 98. The evaluation determiner 90 includes a reproducibility evaluator 94, a mental evaluator 96, and a model processor 97.

The reproducibility evaluator 94 determines a reproducibility evaluation value on the basis of an analysis result regarding the reproducibility of the predetermined body motion by the subject 10. The reproducibility evaluator 94 according to the present embodiment analyzes the reproducibility of a putting motion or a swing motion in golf from a moving image of the putting motion or the swing motion by the subject 10, and determines the reproducibility evaluation value on the basis of the analysis result.

The reproducibility evaluator 94 extracts a predetermined motion feature value as a predetermined parameter regarding the reproducibility on the basis of the information indicating the motion state, and quantifies the reproducibility by the matching degree of the motion feature value over time. The information indicating the motion state mentioned herein is a moving image of a putting motion or a swing motion in golf by the subject 10. The reproducibility evaluator 94 estimates the three-dimensional coordinates of an anatomical feature point from an image of the subject 10 included in the moving image. Examples of the anatomical feature point mentioned herein include not only feature points of body parts such as joints of the subject 10 but also feature points of shoes worn by the subject 10 and feature points of a tool such as a golf club held by the subject 10. The reproducibility evaluator 94 extracts a motion feature value of at least one of the position, the trajectory, and the moving speed of the feature point based on the temporal change of the feature point estimated in the moving image. The reproducibility evaluator 94 quantifies the reproducibility of the motion of the subject 10 on the basis of the matching degree of the motion feature value over time.

The motion feature value extracted by the reproducibility evaluator 94 includes not only a feature value as a time history characteristic that can change over the entire time course of the body motion but also a feature value as an instantaneous characteristic occurring at a specific moment in the body motion.

Examples of the feature value as the time history characteristic include a position and a trajectory of a feature point, a moving speed history, a joint angle, a joint angular velocity, a joint angular acceleration, an upper limb moving speed, an upper limb moving trajectory, a time history of a trunk posture, a stance width, and a face direction. Other examples of the feature value as the time history characteristics include rhythm or speed in a swing sound or a hit sound, a direction of a face obtained from a line of sight, a trajectory of the line of sight, a pupil diameter, a swing speed detected by a motion sensor, an upper limb moving speed, an upper limb moving trajectory, and impact strength. Examples of the feature value as the instantaneous characteristic occurring at a specific moment in the body motion include a maximum swing speed, an impact coefficient, a movable range, a swing sound, a hit sound, and the like.

Various motion feature values extracted by the reproducibility evaluator 94 may be standardized by using a standard deviation or may be variation coefficient values obtained by dividing the standard deviation by an average value. For example, in a case where it is detected by standardization that the trial is a trial in a case where the reproducibility is significantly poor, such as a case of a clear mistake that can occur in execution of a predetermined body motion, such a trial may be processed to be excluded from an evaluation target.

The reproducibility evaluator 94 can determine the reproducibility evaluation value by the matching degree between motions of a plurality of times for each type of the feature value. In addition, the reproducibility evaluator 94 can determine the overall reproducibility evaluation value by a sum of a plurality of feature values or respective reproducibility evaluation values multiplied by weighting factors corresponding to respective degrees of importance. For example, a weighting coefficient α is given to a feature value as an instantaneous characteristic, a weighting coefficient β is given to a feature value as a time history characteristic, and the weighting coefficient α is set to be equal to or larger than the weighting coefficient β (α ≥ β). As a result, the weight for feature values at a moment directly affecting the motion performance can be made larger than that of other times.

In addition, in a case where a plurality of types of feature values are evaluated as feature values as the instantaneous characteristic, the feature values as the instantaneous characteristic may be standardized by multiplying each type of feature value by an individual standardization coefficient in order to commonize the weighting coefficient α given thereto. In addition, in a case where a plurality of types of feature values are evaluated as feature values as the time history characteristic, the feature values as the time history characteristic may be standardized by multiplying each type of feature value by an individual standardization coefficient in order to commonize the weighting coefficient β given thereto. Note that, since there are individual differences in the weighting coefficients α and β, appropriate values as the standardization coefficients for respective feature values, and numerical differences thereof, various feature values may be machine-learned to calculate appropriate values for each subject.

The mental evaluator 96 determines a mental evaluation value on the basis of an analysis result regarding the mental state of the subject 10. The mental evaluator 96 according to the present embodiment executes analysis for quantifying the mental state by a predetermined parameter related to the tendency of the mental state on the basis of information indicating the mental state such as biological information of the subject 10 or answer information input by the subject 10. Examples of the biological information mentioned herein include a heart rate and a blood oxygen level measured by a wearable device such as a smart watch, movement of a line of sight detected by a wearable device such as smart glasses, and the like. The mental evaluator 96 quantifies the mental state by at least one of a fluctuation tendency of the biological information, an answer tendency of the subject 10 to a question regarding the mental information, and a result of a cognitive task for measuring concentration or attention of the subject 10 as a predetermined parameter regarding the tendency of the mental state. The mental evaluator 96 may quantify the mental state by a predetermined parameter related to the tendency of the mental state according to the degree of disturbance stimulus.

The mental evaluator 96 may obtain the mental evaluation value as the sum of an evaluation value as a short-term index and an evaluation value as a medium-to-long-term index. The evaluation value as the short-term index is, for example, a value obtained by multiplying the sum of a value of a mental state indicating the concentration of the subject and a value of a mental state indicating the attention by a predetermined weighting factor. The evaluation value as a medium-to-long-term index is a value obtained by multiplying a value of a mental state indicating motivation of the subject by a predetermined weighting factor. Since there is an individual difference in appropriate values as the weighting factors respectively given to the short-term index and the medium-to-long-term index, various feature values may be machine-learned to determine appropriate values for each subject. In addition, the evaluation value as the short-term index may be an absolute value of a difference between a value of a mental state indicating concentration and attention in a state with good reproducibility and a value of a mental state indicating concentration and attention when a motion is performed under a disturbance stimulus. The evaluation value as the medium-to-long-term index may be an absolute value of a difference between a value of a mental state indicating motivation in a state with good reproducibility and a value of a mental state indicating motivation when performing a motion in daily life.

The model processor 97 performs machine learning by using the reproducibility evaluation value and the mental evaluation value as teacher data to generate a prediction model, and stores the generated prediction model in the evaluation value storage 98 as a personal characteristic of the subject 10. The model processor 97 may generate a prediction model as a regression model in which one of the reproducibility evaluation value and the mental evaluation value is set as an explanatory variable and the other is set as an objective variable. The model processor 97 can estimate the mental evaluation value corresponding to the reproducibility evaluation value indicating a state in which the reproducibility is good on the basis of the prediction model corresponding to the subject 10.

The evaluation value storage 98 stores the reproducibility evaluation value and the mental evaluation value in association with each other as information for each subject 10. The evaluation value storage 98 further stores a prediction model generated on the basis of the reproducibility evaluation value and the mental evaluation value as information for each subject 10.

The result outputter 99 outputs information indicating the relevance between the mental evaluation value and the quality of the reproducibility evaluation value. For example, the result outputter 99 displays the reproducibility evaluation value when the mental evaluation value is low and the reproducibility evaluation value when the mental evaluation value is high side by side, thereby visualizing the relevance between the mental evaluation value and the quality of the reproducibility evaluation value. The result outputter 99 compares the reproducibility evaluation values between the presence and absence of the disturbance stimulus, and displays the reproducibility evaluation value in the presence of the disturbance stimulus and the reproducibility evaluation value in the absence of the disturbance stimulus side by side to visualize the relevance between the quality of the reproducibility evaluation value and the presence or absence of the disturbance stimulus. The result outputter 99 further outputs a result of predicting, on the basis of the prediction model, a mental evaluation value corresponding to a reproducibility evaluation value indicating a state in which the reproducibility is good.

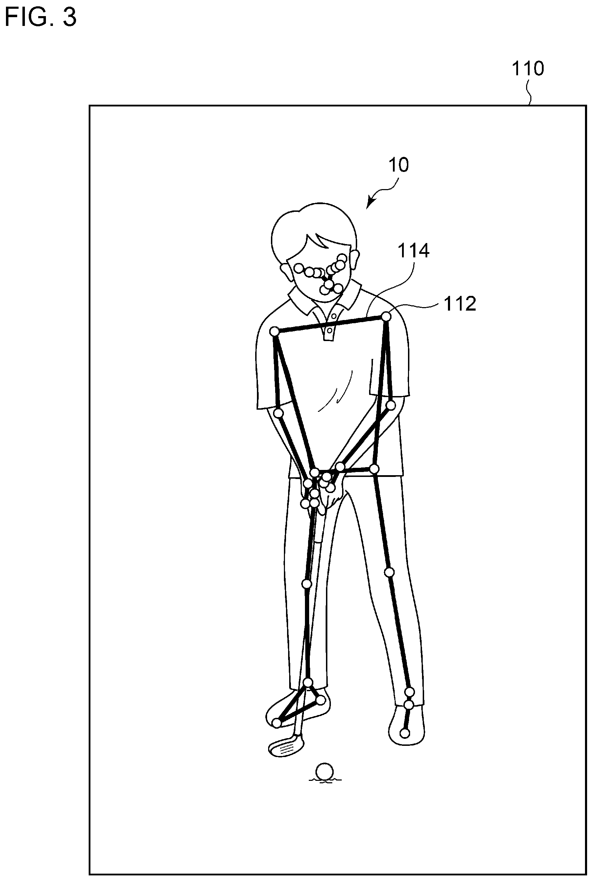

Fig. 3 illustrates a process of estimating a position of an anatomical feature point from an image obtained by photographing the subject putting in golf as an example. The image 110 shows the subject 10 performing a putting motion in golf. By the image processing on the image 110, the positions of main joints and the like, which are anatomical feature points, are estimated as three-dimensional coordinates from the image portion of the body of the subject 10. In Fig. 3, coordinates of the estimated feature points are indicated by a plurality of circles 112. By connecting the plurality of circles 112 indicating the feature points with a thick line 114, the skeleton of the subject 10 is shown in the form of a so-called stick picture. According to the movement of the subject 10 in the image 110, the positions of the circles 112 and the thick line 114 are shown to follow the movement of the feature points. Such positions and movement of the feature points are estimated by the reproducibility evaluator 94, and are extracted as a motion feature value such as a position, a trajectory, and a moving speed of the feature points on the basis of a temporal change of the estimated feature points.

The reproducibility evaluator 94 estimates the three-dimensional coordinates of an anatomical feature point from an image of the subject 10 included in the moving image. The reproducibility evaluator 94 extracts a motion feature value of at least one of the position, the trajectory, and the moving speed of the feature point based on the temporal change of the feature point estimated in the moving image. The reproducibility evaluator 94 quantifies the reproducibility of the motion of the subject 10 on the basis of the matching degree of the motion feature value over time.

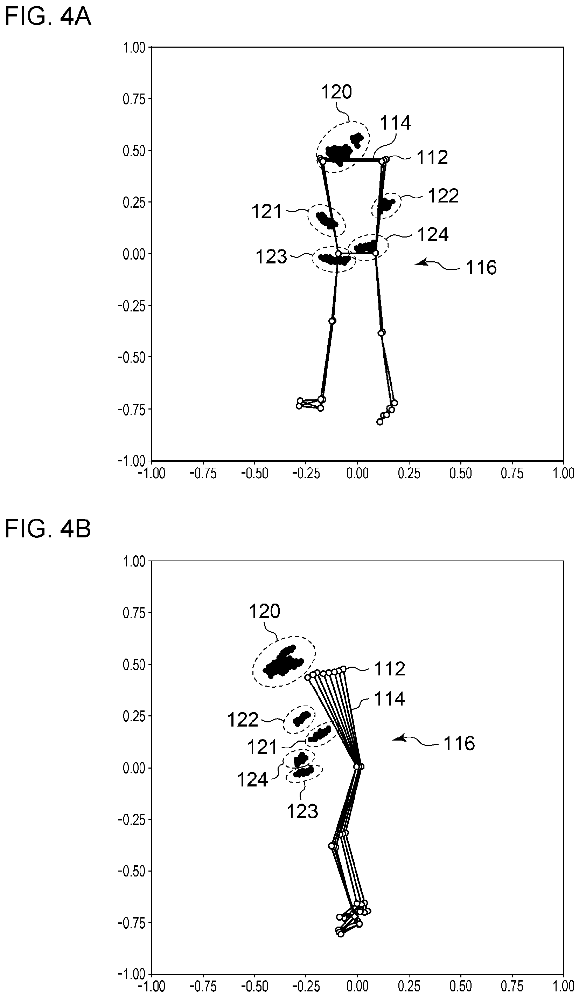

Fig. 4 illustrates an example in which the positions of a plurality of feature points in a case where the same putting motion is repeated three times are indicated in two-dimensional coordinates. Fig. 4A is an image obtained by photographing the front side of the subject 10, and Fig. 4B is an image obtained by photographing the left side of the subject 10. From these images, a stick picture 116 formed by connecting the plurality of circles 112, which are anatomical feature points, by a thick line 114, and estimated by a known motion analysis technique is illustrated. The stick picture 116 is arranged such that the center of the pelvis is located at the origin of the horizontal axis and the vertical axis, and each feature point is shown in a relative position with respect to the origin.

A point group surrounded by a broken line 120 indicates the positions of the feature points of the face of the subject 10 in a plurality of putting motions, and the distribution range of the point group indicates the movement range of the feature points. Similarly, a point group surrounded by a broken line 121 indicates the position and movement of the right elbow of the subject 10, and a point group surrounded by a broken line 122 indicates the position and movement of the left elbow of the subject 10. A point group surrounded by a broken line 123 indicates the position and movement of the right wrist of the subject 10, and a point group surrounded by a broken line 124 indicates the position and movement of the left wrist of the subject 10.

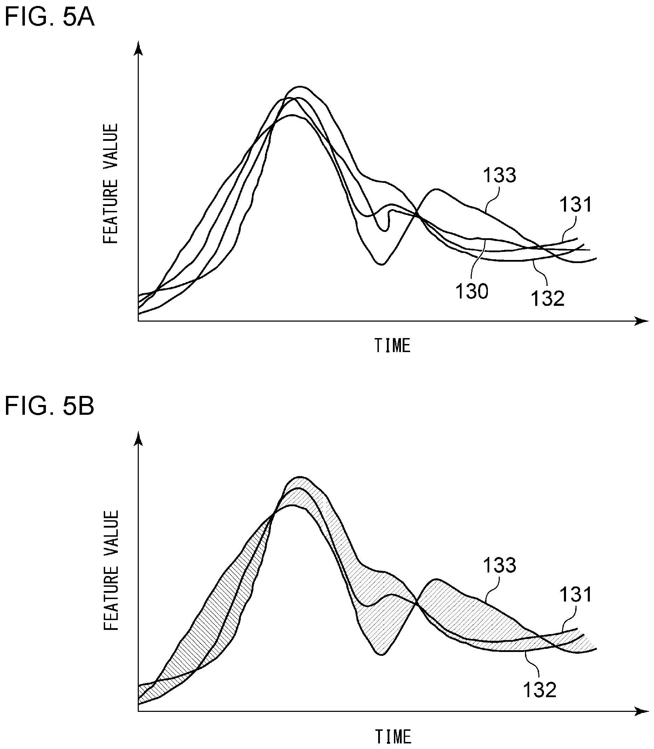

Fig. 5 is a graph illustrating a time history characteristic of a motion feature value in a case where the same putting motion is repeated three times. In the graph, the vertical axis represents the feature value, and the horizontal axis represents the time. The motion feature value mentioned herein is not limited to the information of the positions of the anatomical feature points illustrated in Figs. 3 and 4, and may be another value such as a frequency or a volume obtained by acoustic analysis as long as the motion feature value is information indicating the motion. Alternatively, the motion feature value may be a pupil diameter, a trajectory of a line of sight, or the like obtained by analyzing information of the line of sight, or may be a motion speed, a motion acceleration, or an amplitude strength of an impact.

In the example of Fig. 5A, the motion reproducibility is evaluated on the basis of the average of the differences from the average data of motions. A line 131 indicates the feature value in the first motion, a line 132 indicates the feature value in the second motion, a line 133 indicates the feature value in the third motion, and a line 130 indicates an average value of the motion feature values. The lines 131 to 133 indicating three motions are arranged on the time axis on the basis of a predetermined timing, for example, a moment at which a putter impacts a ball.

The absolute value of the difference between the feature value for each time point in one motion indicated by the lines 131 to 133 and the feature value for each time point in the motion of the average data indicated by the line 130 is averaged over time to calculate the average value of the feature values of one motion. An average value of the feature values of one motion is further averaged for three motions to calculate an evaluation value of motion reproducibility. A smaller evaluation value indicates a relatively higher motion reproducibility, and a larger evaluation value indicates a relatively lower motion reproducibility.

When the variation in the motion of the three motions is smaller, the evaluation value becomes smaller, and the lines 131, 132, and 133 converge to the line 130 indicating the average value, which indicates that the motion reproducibility is high. When the variation in the motion of the three motions is larger, the evaluation value becomes larger, and the lines 131, 132, and 133 do not converge to the line 130 and the distance therebetween increases, which indicates that the motion reproducibility is low.

In addition, one body motion may partially include a portion with high motion reproducibility and a portion with low motion reproducibility over time. For example, on the timeline, a portion with a narrow mutual interval between the lines 131, 132, and 133 indicates a motion portion with relatively high motion reproducibility, and a portion with a wide mutual interval between the lines 131, 132, and 133 indicates a motion portion with relatively low motion reproducibility.

In the example of Fig. 5B, the motion reproducibility is evaluated by using the sum of products of the differences of the motion feature values in the time history. The line 131 indicates the feature value in the first motion, the line 132 indicates the feature value in the second motion, and the line 133 indicates the feature value in the third motion. In Fig. 5B, hatching is applied to a range from the minimum feature value to the maximum feature value for each time indicated by the lines 131 to 133. A larger area of the hatched region indicates lower operation reproducibility, and a smaller area of the hatched region indicates higher operation reproducibility. The area of the hatched region is obtained by a piecewise quadrature method in which a difference between the maximum feature value and the minimum feature value at a time k is integrated, and thus the evaluation value of the motion reproducibility is obtained. A smaller evaluation value indicates a relatively higher motion reproducibility, and a larger evaluation value indicates a relatively lower motion reproducibility.

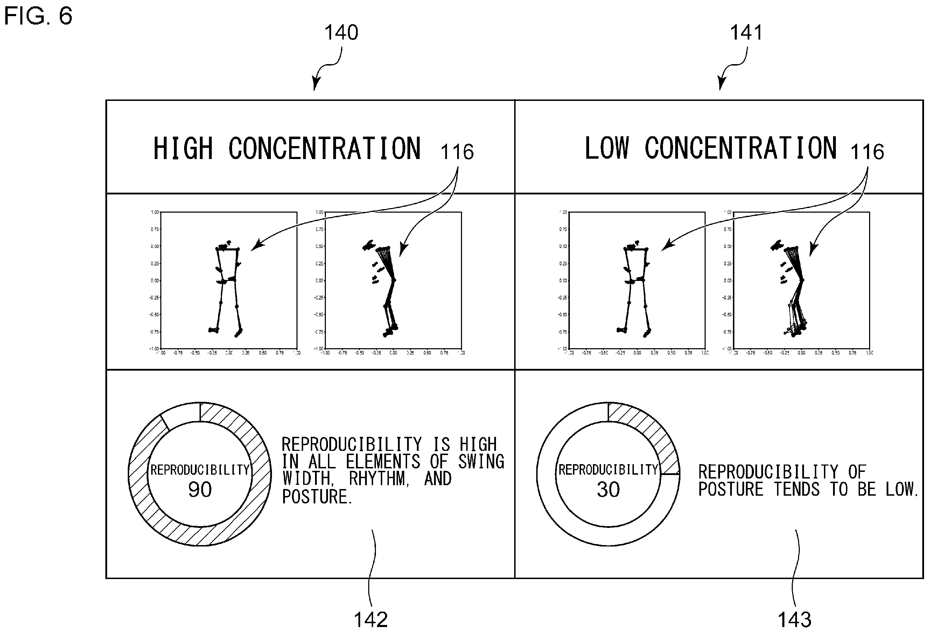

Fig. 6 illustrates a screen example of comparing and displaying operation reproducibility in a case where the mental state is good and operation reproducibility in a case where the mental state is bad. In a left column 140, a reproducibility evaluation value such as "REPRODUCIBILITY 90" is displayed as characters with a pie chart as the motion reproducibility in a case where the mental state is good. In a right column 141, a reproducibility evaluation value such as "REPRODUCIBILITY 30" is displayed as characters with a pie chart as the motion reproducibility in a case where the mental state is bad. In addition, animation of the stick picture 116 indicating the motion reproducibility in a case where the mental state is good is displayed in the left column 140, and animation of the stick picture 116 indicating the motion reproducibility in a case where the mental state is bad is displayed in the right column 141. In the left column 140, a comment exemplifying a feature value indicating a characteristic evaluation value in a case where the mental state is good is displayed. In the right column 141, a comment exemplifying a feature value indicating a characteristic evaluation value in a case where the mental state is bad is displayed.

### Second Embodiment

The second embodiment is different from the first embodiment in which the present disclosure is applied to the relevance between the mental state and the reproducibility of the putting motion or the swing motion of golf, in that the subject 10 applies the present disclosure to the relevance between the mental state and the reproducibility of the running form. Hereinafter, differences from the first embodiment will be mainly described, and description of common points will be omitted.

In the example of the second embodiment, the subject 10 performing a running motion is photographed by the camera function of the information terminal 12, and the photographed image is transmitted to the mental/physical state evaluation server 50 as information indicating the motion state. The subject 10 wears, for example, on a wrist, a foot, or the like thereof, the measurement device 20 such as a smart watch or a foot pod that acquires biological information, for example, information indicating a mental state such as a heart rate, a blood oxygen level, and a pupil response, and information indicating a motion state such as a rhythm and speed of swing of arms.

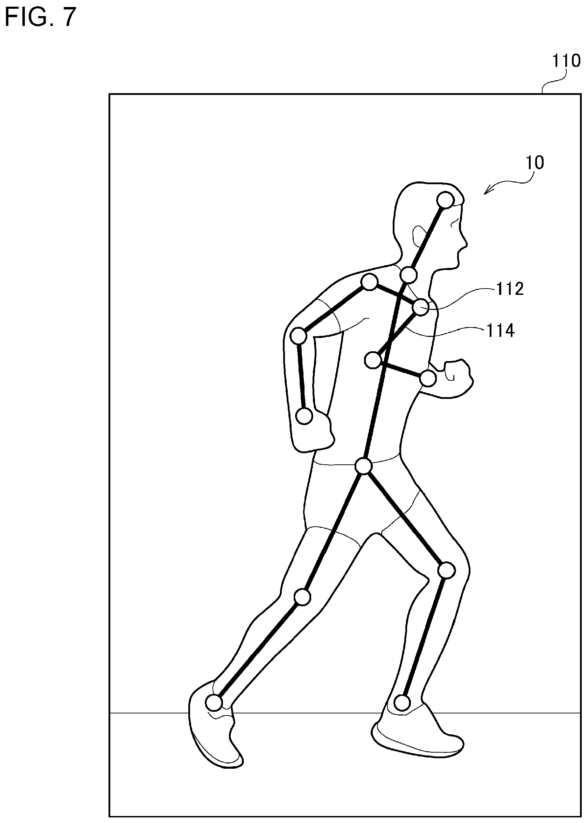

Fig. 7 illustrates a process of estimating a position of an anatomical feature point from an image obtained by photographing a running subject. The image 110 shows the subject 10 performing a running motion. By the image processing on the image 110, the positions of main joints and the like, which are anatomical feature points, are estimated as three-dimensional coordinates from the image portion of the body of the subject 10. In Fig. 7, coordinates of the estimated feature points are indicated by a plurality of circles 112. By connecting the plurality of circles 112 indicating the feature points with a thick line 114, the skeleton of the subject 10 is shown in the form of a so-called stick picture. According to the movement of the subject 10 in the image 110, the positions of the circles 112 and the thick line 114 are shown to follow the movement of the feature points.

This will be described with reference to Fig. 2. The measurement device 20 is, for example, a wearable device such as a smart watch worn on the wrist of the subject, smart glasses that are worn on the eyes like glasses or sunglasses, or a foot pod worn on the ankle or a shoe. In a case where the measurement device 20 is worn on the ankle or the shoe, the motion detector 26 or the motion acquirer 76 acquires information such as acceleration, cadence, and stride length of the motion of the leg detected by a motion sensor such as a 9-axis sensor. The moving image acquirer 32 or the moving image acquirer 72 acquires a video obtained by capturing the subject 10 performing a running motion. The sound acquirer 34 or the sound acquirer 74 acquires a breathing sound, a landing sound, and the like during the running motion of the subject 10.

The reproducibility evaluator 94 analyzes the reproducibility of the motion such as the running form, the cadence, and the stride length from a moving image of the running motion of the subject 10 and the motion information acquired by the motion detector 26 or the motion acquirer 76, and determines the reproducibility evaluation value on the basis of the analysis result. The reproducibility evaluator 94 extracts a predetermined motion feature value as a predetermined parameter regarding the reproducibility on the basis of the information indicating the motion state, and quantifies the reproducibility by the matching degree of the motion feature value over time. The information indicating the motion state mentioned herein is a moving image of the running motion of the subject 10 or information of the motion acquired by the motion detector 26 or the motion acquirer 76. The reproducibility evaluator 94 estimates the three-dimensional coordinates of an anatomical feature point from an image of the subject 10 included in the moving image. Examples of the anatomical feature point mentioned herein include not only feature points of body parts such as joints of the subject 10 but also feature points of shoes worn by the subject 10. Positions and movement of the feature points as illustrated in Fig. 7 are estimated by the reproducibility evaluator 94, and are extracted as a motion feature value such as a position, a trajectory, and a moving speed of the feature points on the basis of a temporal change of the estimated feature points.

Examples of the feature value as the time history characteristic extracted by the reproducibility evaluator 94 include a position and a trajectory of a feature point, a moving speed history, a joint angle, a joint angular velocity, a joint angular acceleration, an upper limb moving speed, an upper limb moving trajectory, a time history of a trunk posture, a cadence, a stride length, and a face direction. Other examples of the feature value as the time history characteristics include rhythm or speed of an arm swing or landing in a breathing sound or a landing sound, a direction of a face obtained from a line of sight, a trajectory of the line of sight, a pupil diameter, a swing speed of an arm or a leg detected by a motion sensor, an upper limb moving speed, an upper limb moving trajectory, and a running pace. The running pace may be calculated on the basis of the cadence and the stride length, or may be calculated on the basis of a running history in which position information received by the measurement device 20 from a satellite positioning system such as a global positioning system (GPS) is recorded. Examples of the feature value as the instantaneous characteristic occurring at a specific moment in the body motion include a maximum swing speed of the arm or the leg, a landing impact, a movable range, strength of a breathing sound, landing, and kicking, and the like.

Also in the second embodiment, the relevance between the quality of the mental state and the quality of the motion reproducibility is visualized, and the reproducibility evaluation values corresponding to a plurality of situations having different mental states are compared and displayed. Therefore, for example, in a case where the running performance has deteriorated, the subject 10 can find a clue to improvement in the mental state by oneself.

### Third Embodiment

The third embodiment is different from the first and second embodiments in which the present disclosure is applied to the relevance between the mental state and the reproducibility of the putting motion or the swing motion of golf or the running motion, in that the subject 10 applies the present disclosure to the relevance between the mental state and the reproducibility of a trick in skateboarding. Hereinafter, differences from the first and second embodiments will be mainly described, and description of common points will be omitted.

In the example of the third embodiment, the subject 10 performing a trial of a motion of a skill called a trick in skateboarding is photographed by the camera function of the information terminal 12, and the photographed image is transmitted to the mental/physical state evaluation server 50 as information indicating the motion state. The subject 10 wears, for example, on a wrist, a helmet, a knee pad, an ankle, a skateboard, or the like thereof, the measurement device 20 such as a smart watch or the like that acquires biological information, for example, information indicating a mental state such as a heart rate, a blood oxygen level, and a pupil response, and information indicating a motion state such as a rotational angle, an altitude, a speed, and an in-air time.

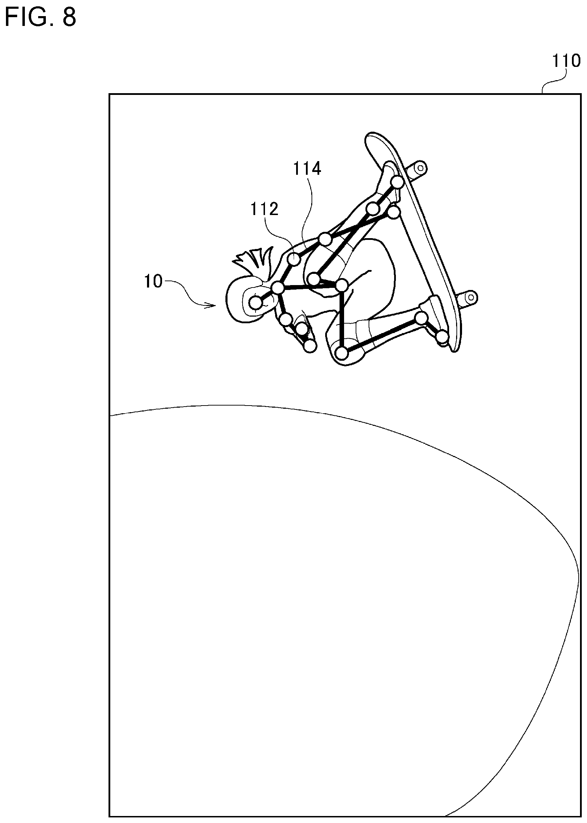

Fig. 8 illustrates a process of estimating a position of an anatomical feature point from an image obtained by photographing a subject performing a trial of a trick. The image 110 shows the subject 10 performing a trick motion. By the image processing on the image 110, the positions of main joints and the like, which are anatomical feature points, are estimated as three-dimensional coordinates from the image portion of the body of the subject 10. In Fig. 8, coordinates of the estimated feature points are indicated by a plurality of circles 112. By connecting the plurality of circles 112 indicating the feature points with a thick line 114, the skeleton of the subject 10 is shown in the form of a so-called stick picture. According to the movement of the subject 10 in the image 110, the positions of the circles 112 and the thick line 114 are shown to follow the movement of the feature points.

This will be described with reference to Fig. 2. The measurement device 20 is, for example, a wearable device such as a smart watch worn on the wrist of the subject, smart glasses that are worn on the eyes like glasses or sunglasses, or a sensor worn on a helmet, a knee pad, an ankle, or a skateboard. In a case where the measurement device 20 is worn on the helmet, the knee pad, the ankle, the skateboard, or the like, the motion detector 26 or the motion acquirer 76 acquires information such as the rotational angle, altitude, speed, in-air time, and the like detected by a motion sensor such as a 9-axis sensor. The moving image acquirer 32 or the moving image acquirer 72 acquires a video obtained by capturing the subject 10 performing a trial of a trick motion. The sound acquirer 34 or the sound acquirer 74 acquires a breathing sound, a traveling sound, a landing sound, and the like during the trick motion of the subject 10.

The reproducibility evaluator 94 analyzes the reproducibility of the trick based on the degree of deviation of the posture or the motion from a trick in a specific trial on the basis of a moving image of the trick motion of the subject 10 and the motion information acquired by the motion detector 26 or the motion acquirer 76, and determines the reproducibility evaluation value on the basis of the analysis result. The specific trial mentioned herein may be, for example, a moving image or motion information of a past trial performed by the subject 10 themself. Alternatively, instead of the trial of the subject 10 themself, for example, a moving image or motion information of a past trial performed by another person such as an olympic athlete, or a moving image or motion information of a motion of a stick picture performing, as a model, an ideal trick by being operated or set by a person.

The reproducibility evaluator 94 extracts a predetermined motion feature value as a predetermined parameter regarding the reproducibility on the basis of the information indicating the motion state, and quantifies the reproducibility by the matching degree of the motion feature value over time. The level of the reproducibility of the trick may be defined as the success rate of the trick. The information indicating the motion state mentioned herein is a moving image of the trial of the subject 10 or information of the motion acquired by the motion detector 26 or the motion acquirer 76. The reproducibility evaluator 94 estimates the three-dimensional coordinates of an anatomical feature point from an image of the subject 10 included in the moving image. Examples of the anatomical feature point mentioned herein include not only feature points of body parts such as joints of the subject 10 but also feature points of shoes worn by the subject 10, and tools such as skateboards. Positions and movement of the feature points as illustrated in Fig. 8 are estimated by the reproducibility evaluator 94, and are extracted as a motion feature value such as a position, a trajectory, and a moving speed of the feature points on the basis of a temporal change of the estimated feature points.

Examples of the feature value as the time history characteristic extracted by the reproducibility evaluator 94 include a position and a trajectory of a feature point, a moving speed, a moving direction, a body direction, a posture, a rotational angle, a distance from a section, an altitude, an in-air time, a joint angle, a joint angular velocity, a joint angular acceleration, an upper limb moving speed, an upper limb moving trajectory, a time history of a trunk posture, and a face direction. Other examples of the feature value as the time history characteristic include a timing of a trick in a traveling sound or a landing sound, a portion of a skateboard that comes into contact with a section, a direction of a face obtained from a line of sight, a trajectory of the line of sight, a pupil diameter, a direction and acceleration of a trick motion detected by a motion sensor, a moving speed, an upper limb moving speed, and an upper limb moving trajectory. Examples of the feature value as the instantaneous characteristic occurring at a specific moment in the body motion include a speed and strength of a jump, a landing impact, and a movable range.

Also in the second embodiment, the relevance between the quality of the mental state and the quality of the motion reproducibility is visualized, and the reproducibility evaluation values corresponding to a plurality of situations having different mental states are compared and displayed. Therefore, for example, in a case where the success rate of a trick has deteriorated, the subject 10 can find a clue to improvement in the mental state by oneself.

The present disclosure is not limited to the above-described embodiments, and each configuration can be appropriately changed without departing from the gist of the present disclosure.

In each of the embodiments described above, an example in which the present disclosure is applied to the relevance between the mental state and the reproducibility of a putting motion and a swing motion in golf, a running motion, and a trick in skateboarding has been described. In modification examples, the present disclosure may be applied to the motion reproducibility of other body motions, for example, other sports such as a swing in baseball, a kick in soccer, martial arts, a shooting motion in a ball game such as basketball, scoring sports such as figure skating, dance, and gymnastics, and track and field sports such as jumping and throwing, or may be applied to the reproducibility of musical instrument performance such as a piano.

## Claims

1. A mental/physical state evaluation system (100) comprising:
a reproducibility evaluator (94) configured to determine a reproducibility evaluation value quantifying high or low reproducibility on a basis of an analysis result regarding reproducibility of a predetermined body motion of a subject;
wherein the reproducibility evaluator (94) performs analysis for quantifying the reproducibility by using a predetermined parameter related to the reproducibility of the predetermined body motion on a basis of information indicating a motion state, and determines the reproducibility evaluation value on the basis of the analysis result thereof;
a mental evaluator (96) configured to determine a mental evaluation value quantifying good or bad mental state on a basis of an analysis result regarding a mental state of the subject;
wherein the mental evaluator (96) performs analysis for quantifying the mental state by using a predetermined parameter related to the mental state on a basis of information indicating the mental state, and determines the mental evaluation value on the basis of the analysis result thereof;
an evaluation value storage (98) configured to store the reproducibility evaluation value and the mental evaluation value in association with each other;
a result outputter (99) configured to output information indicating relevance between the good or bad mental state based on the mental evaluation value and the high or low reproducibility based on the reproducibility evaluation value by displaying the reproducibility evaluation value and the mental evaluation value; and
a motion information acquirer (70) configured to acquire information indicating the motion state of the subject repeatedly performing the predetermined body motion;
wherein the motion information acquirer includes a moving image acquirer (32), a sound acquirer (34), and a motion detector (36); and
a mental information acquirer (80) configured to acquire information indicating the mental state of the subject,
wherein the mental information acquirer (80) includes a disturbance information acquirer (83);
wherein the disturbance information is obtained by the subject selecting and inputting an item of external stimulus and the subject subjectively determining the degree of stimulus and inputting a numerical value to the disturbance information acquirer.

2. The mental/physical state evaluation system (100) according to claim 1,
wherein the mental information acquirer (80) further includes any one or more of: a biological information acquirer (81), a line-of-sight acquirer (82), a question answer acquirer (84), a task result acquirer (85), and an action information acquirer (86).

3. The mental/physical state evaluation system (100) according to claim 1 or 2, wherein the reproducibility evaluator (94) extracts a predetermined motion feature value as the predetermined parameter related to the reproducibility on the basis of the information indicating the motion state, and quantifies the reproducibility in accordance with a degree of matching of the motion feature value over time.

4. The mental/physical state evaluation system (100) according to any one of claims 1 to 3, wherein the mental evaluator (96) quantifies the mental state by using, as the predetermined parameter related to the mental state, at least one of a fluctuation of predetermined biological information of the subject, an answer of the subject to a question regarding mental information, and a result of a cognitive task for measuring concentration or attention of the subject.

5. The mental/physical state evaluation system (100) according to any one of claims 1 to 4,
wherein the mental information acquirer (80) further acquires information indicating a degree of disturbance stimulus that is possible to affect the mental state of the subject, and
wherein the mental evaluator (96) quantifies the mental state by using the predetermined parameter related to the mental state corresponding to the degree of disturbance stimulus.

6. The mental/physical state evaluation system (100) according to any one of claims 1 to 5,
wherein the evaluation value storage (98) further stores a prediction model generated on a basis of the reproducibility evaluation value and the mental evaluation value, and
wherein the result outputter (99) further outputs a result of predicting, on the basis of the prediction model, the mental evaluation value corresponding to the reproducibility evaluation value indicating a state in which the reproducibility is high.

7. A computer-implemented mental/physical state evaluation method comprising:
determining a reproducibility evaluation value quantifying high or low reproducibility on a basis of an analysis result regarding reproducibility of a predetermined body motion of a subject;
performing analysis for quantifying the reproducibility by using a predetermined parameter related to the reproducibility of the predetermined body motion on a basis of the information indicating the motion state, and determining the reproducibility evaluation value on the basis of the analysis result thereof;
determining a mental evaluation value quantifying good or bad mental state on a basis of an analysis result regarding a mental state of the subject;
performing analysis for quantifying the mental state by using a predetermined parameter related to the mental state on a basis of the information indicating the mental state, and determining the mental evaluation value on the basis of the analysis result thereof;
storing the reproducibility evaluation value and the mental evaluation value in association with each other; and
outputting information indicating relevance between the good or bad mental state based on the mental evaluation value and the high or low reproducibility based on the reproducibility evaluation value by displaying the reproducibility evaluation value and the mental evaluation value; and
acquiring information indicating the motion state of the subject repeatedly performing the predetermined body motion using a motion information acquirer (70);
wherein the motion information acquirer includes a moving image acquirer (32), a sound acquirer (34), and a motion detector (36); and
acquiring information indicating the mental state of the subject using a mental information acquirer (80);
wherein the mental information acquirer (80) includes a disturbance information acquirer (83);
wherein the disturbance information is obtained by the subject selecting and inputting an item of external stimulus and the subject subjectively determining the degree of stimulus and inputting a numerical value to the disturbance information acquirer.

## Patentansprüche

1. Bewertungssystem (100) für einen mentalen/physischen Zustand, das Folgendes umfasst:
einen Reproduzierbarkeitsbewerter (94), der dafür konfiguriert ist, einen Reproduzierbarkeitsbewertungswert zu bestimmen, der eine hohe oder niedrige Reproduzierbarkeit auf einer Grundlage eines Analyseergebnisses hinsichtlich der Reproduzierbarkeit einer vorbestimmten Körperbewegung eines Subjekts quantifiziert;
wobei der Reproduzierbarkeitsbewerter (94) eine Analyse zum Quantifizieren der Reproduzierbarkeit durch Verwenden eines vorbestimmten Parameters durchführt, der mit der Reproduzierbarkeit der vorbestimmten Körperbewegung zusammenhängt, auf einer Grundlage von Informationen, die einen Bewegungszustand anzeigen, und den Reproduzierbarkeitsbewertungswert auf der Grundlage des Analyseergebnisses derselben bestimmt;
einen Mentalbewerter (96), der dafür konfiguriert ist, einen Mentalbewertungswert zu bestimmen, der einen guten oder schlechten mentalen Zustand auf einer Grundlage eines Analyseergebnisses hinsichtlich des mentalen Zustands des Subjekts quantifiziert;
wobei der Mentalbewerter (96) eine Analyse zum Quantifizieren des mentalen Zustands durch Verwenden eines vorbestimmten Parameters durchführt, der mit dem mentalen Zustand zusammenhängt, auf einer Grundlage von Informationen, die den mentalen Zustand anzeigen, und den Mentalbewertungswert auf der Grundlage des Analyseergebnisses desselben bestimmt;
einen Bewertungswertspeicher (98), der dafür konfiguriert ist, den Reproduzierbarkeitsbewertungswert und den Mentalbewertungswert in Verbindung miteinander zu speichern;
einen Ergebnisausgeber (99), der dafür konfiguriert ist, Informationen auszugeben, die eine Relevanz zwischen dem guten oder schlechten mentalen Zustand auf Grundlage des Mentalbewertungswertes und der hohen oder niedrigen Reproduzierbarkeit auf Grundlage des Reproduzierbarkeitsbewertungswertes anzeigen, durch Anzeigen des Reproduzierbarkeitsbewertungswertes und des Mentalbewertungswertes; und
einen Bewegungsdatenerfasser (70), der dafür konfiguriert ist, Informationen zu erfassen, die den Bewegungszustand des Subjekts anzeigen, das wiederholt die vorbestimmte Körperbewegung ausführt;
wobei der Bewegungsdatenerfasser einen Bewegtbilderfasser (32), einen Schallerfasser (34) und einen Bewegungsdetektor (36) einschließt; und
einen Mentalinformationserfasser (80), der dafür konfiguriert ist, Informationen zu erfassen, die den mentalen Zustand des Subjekts anzeigen,
wobei der Mentalinformationserfasser (80) einen Störungsinformationserfasser (83) einschließt;
wobei die Störungsinformationen dadurch gewonnen werden, dass das Subjekt ein externes Reizelement auswählt und eingibt und das Subjekt subjektiv den Grad des Reizes bestimmt und einen numerischen Wert in den Störungsinformationserfasser eingibt.

2. Bewertungssystem (100) für einen mentalen/physischen Zustand nach Anspruch 1,
wobei der Mentalinformationserfasser (80) weiter eines oder mehrere von Folgendem einschließt: einen biologischen Informationserfasser (81), einen Sichtlinien-Erfasser (82), einen Frage-Antwort-Erfasser (84), einen Aufgabe-Ergebnis-Erfasser (85) und einen Handlungsinformationserfasser (86).

3. Bewertungssystem (100) für einen mentalen/physischen Zustand nach Anspruch 1 oder 2, wobei der Reproduzierbarkeitsbewerter (94) auf der Grundlage der Informationen, die den Bewegungszustand anzeigen, einen vorbestimmten Bewegungsmerkmalswert als den vorbestimmten Parameter, der mit der Reproduzierbarkeit zusammenhängt, extrahiert und die Reproduzierbarkeit entsprechend einem Grad der Übereinstimmung des Bewegungsmerkmalswertes im Laufe der Zeit quantifiziert.

4. Bewertungssystem (100) für einen mentalen/physischen Zustand nach einem der Ansprüche 1 bis 3, wobei der Mentalbewerter (96) den mentalen Zustand quantifiziert, durch Verwenden, als den vorbestimmten Parameter, der mit dem mentalen Zustand zusammenhängt, mindestens eines von einer Schwankung vorbestimmter biologischer Informationen des Subjekts, einer Antwort des Subjekts auf eine Frage hinsichtlich mentaler Informationen und einem Ergebnis einer kognitiven Aufgabe zum Messen von Konzentration oder Aufmerksamkeit des Subjekts.

5. Bewertungssystem (100) für einen mentalen/physischen Zustand nach einem der Ansprüche 1 bis 4,
wobei der Mentalinformationserfasser (80) weiter Informationen erfasst, die einen Grad eines Störreizes anzeigen, der möglicherweise den mentalen Zustand des Subjekts beeinflusst, und
wobei der Mentalbewerter (96) den mentalen Zustand durch Verwenden des vorbestimmten Parameters quantifiziert, der mit dem mentalen Zustand zusammenhängt, der dem Grad eines Störreizes entspricht.

6. Bewertungssystem (100) für einen mentalen/physischen Zustand nach einem der Ansprüche 1 bis 5,
wobei der Bewertungswertspeicher (98) weiter ein auf einer Grundlage des Reproduzierbarkeitsbewertungswertes und des Mentalbewertungswertes erzeugtes Vorhersagemodell speichert und
wobei der Ergebnisausgeber (99) weiter ein Ergebnis ausgibt, das auf der Grundlage des Vorhersagemodells den Mentalbewertungswert vorhersagt, der dem Reproduzierbarkeitsbewertungswert entspricht, der einen Zustand anzeigt, in dem die Reproduzierbarkeit hoch ist.

7. Computerimplementiertes Bewertungsverfahren für einen mentalen/physischen Zustand, das Folgendes umfasst:
Bestimmen eines Reproduzierbarkeitsbewertungswertes, der eine hohe oder niedrige Reproduzierbarkeit auf der Grundlage eines Analyseergebnisses hinsichtlich der Reproduzierbarkeit einer vorbestimmten Körperbewegung eines Subjekts quantifiziert;
Durchführen einer Analyse zum Quantifizieren der Reproduzierbarkeit unter Verwendung eines vorbestimmten Parameters, der mit der Reproduzierbarkeit der vorbestimmten Körperbewegung zusammenhängt, auf einer Grundlage der Informationen, die den Bewegungszustand anzeigen, und Bestimmen des Reproduzierbarkeitsbewertungswertes auf der Grundlage des Analyseergebnisses derselben;
Bestimmen eines Mentalbewertungswertes, der einen guten oder schlechten mentalen Zustand auf einer Grundlage eines Analyseergebnisses hinsichtlich des mentalen Zustands des Subjekts quantifiziert;
Durchführen einer Analyse zum Quantifizieren des mentalen Zustands durch Verwenden eines vorbestimmten Parameters, der mit dem mentalen Zustand zusammenhängt, auf einer Grundlage der Informationen, die den mentalen Zustand anzeigen, und Bestimmen des Mentalbewertungswertes auf der Grundlage des Analyseergebnisses derselben;
Speichern des Reproduzierbarkeitsbewertungswertes und des Mentalbewertungswertes in Verbindung miteinander und
Ausgeben von Informationen, die eine Relevanz zwischen dem guten oder schlechten mentalen Zustand auf Grundlage des Mentalbewertungswertes und der hohen oder niedrigen Reproduzierbarkeit auf Grundlage des Reproduzierbarkeitsbewertungswertes anzeigen, durch Anzeigen des Reproduzierbarkeitsbewertungswertes und des Mentalbewertungswertes; und
Erfassen von Informationen, die den Bewegungszustand des Subjekts anzeigen, das wiederholt die vorbestimmte Körperbewegung ausführt, unter Verwendung eines Bewegungsdatenerfassers (70);
wobei der Bewegungsdatenerfasser einen Bewegtbilderfasser (32), einen Schallerfasser (34) und einen Bewegungsdetektor (36) einschließt; und
Erfassen von Informationen, die den mentalen Zustand des Subjekts anzeigen, unter Verwendung eines Mentalinformationserfassers (80);
wobei der Mentalinformationserfasser (80) einen Störungsinformationserfasser (83) einschließt;
wobei die Störungsinformationen dadurch gewonnen werden, dass das Subjekt ein externes Reizelement auswählt und eingibt und das Subjekt subjektiv den Grad des Reizes bestimmt und einen numerischen Wert in den Störungsinformationserfasser eingibt.

## Revendications

1. Système d'évaluation d'état mental/physique (100) comprenant :
un évaluateur de reproductibilité (94) configuré pour déterminer une valeur d'évaluation de reproductibilité quantifiant une reproductibilité élevée ou faible sur la base d'un résultat d'analyse concernant la reproductibilité d'un mouvement corporel prédéterminé d'un sujet ;
dans lequel l'évaluateur de reproductibilité (94) effectue une analyse pour quantifier la reproductibilité en utilisant un paramètre prédéterminé lié à la reproductibilité du mouvement corporel prédéterminé sur la base d'informations indiquant un état de mouvement, et détermine la valeur d'évaluation de reproductibilité sur la base du résultat d'analyse de celle-ci ;
un évaluateur mental (96) configuré pour déterminer une valeur d'évaluation mentale quantifiant un bon ou un mauvais état mental sur la base d'un résultat d'analyse concernant un état mental du sujet ;
dans lequel l'évaluateur mental (96) effectue une analyse pour quantifier l'état mental en utilisant un paramètre prédéterminé lié à l'état mental sur la base d'informations indiquant l'état mental, et détermine la valeur d'évaluation mentale sur la base du résultat d'analyse de celle-ci ;
un stockage de valeur d'évaluation (98) configuré pour stocker la valeur d'évaluation de reproductibilité et la valeur d'évaluation mentale en association l'une avec l'autre ;
un générateur de résultats (99) configuré pour délivrer en sortie des informations indiquant la pertinence entre le bon ou mauvais état mental sur la base de la valeur d'évaluation mentale et la reproductibilité élevée ou faible sur la base de la valeur d'évaluation de reproductibilité en affichant la valeur d'évaluation de reproductibilité et la valeur d'évaluation mentale ; et
un acquéreur d'informations de mouvement (70) configuré pour acquérir des informations indiquant l'état de mouvement du sujet effectuant de manière répétée le mouvement corporel prédéterminé ;
dans lequel l'acquéreur d'informations de mouvement inclut un acquéreur d'images animées (32), un acquéreur de son (34) et un détecteur de mouvement (36) ; et
un acquéreur d'informations mentales (80) configuré pour acquérir des informations indiquant l'état mental du sujet,
dans lequel l'acquéreur d'informations mentales (80) inclut un acquéreur d'informations de perturbation (83) ;
dans lequel les informations de perturbation sont obtenues par la sélection et la saisie, par le sujet, d'un élément de stimulus externe, et par la détermination subjective, par le sujet, du degré de stimulus et la saisie, par le sujet, d'une valeur numérique dans l'acquéreur d'informations de perturbation.

2. Système d'évaluation d'état mental/physique (100) selon la revendication 1,
dans lequel l'acquéreur d'informations mentales (80) inclut en outre un ou plusieurs parmi : un acquéreur d'informations biologiques (81), un acquéreur de ligne de visée (82), un acquéreur de réponse aux questions (84), un acquéreur de résultat de tâche (85) et un acquéreur d'informations d'action (86).

3. Système d'évaluation d'état mental/physique (100) selon la revendication 1 ou 2, dans lequel l'évaluateur de reproductibilité (94) extrait une valeur de caractéristique de mouvement prédéterminée comme paramètre prédéterminé lié à la reproductibilité sur la base des informations indiquant l'état de mouvement, et quantifie la reproductibilité en fonction d'un degré de correspondance de la valeur de caractéristique de mouvement au fil du temps.

4. Système d'évaluation d'état mental/physique (100) selon l'une quelconque des revendications 1 à 3, dans lequel l'évaluateur mental (96) quantifie l'état mental en utilisant, comme paramètre prédéterminé lié à l'état mental, au moins l'un parmi une fluctuation d'informations biologiques prédéterminées du sujet, une réponse du sujet à une question concernant des informations mentales et un résultat d'une tâche cognitive pour mesurer la concentration ou l'attention du sujet.

5. Système d'évaluation d'état mental/physique (100) selon l'une quelconque des revendications 1 à 4,
dans lequel l'acquéreur d'informations mentales (80) acquiert en outre des informations indiquant un degré de stimulus perturbateur qui est susceptible d'affecter l'état mental du sujet, et
dans lequel l'évaluateur mental (96) quantifie l'état mental en utilisant le paramètre prédéterminé lié à l'état mental correspondant au degré de stimulus perturbateur.

6. Système d'évaluation d'état mental/physique (100) selon l'une quelconque des revendications 1 à 5,
dans lequel le stockage de valeur d'évaluation (98) stocke en outre un modèle de prédiction généré sur la base de la valeur d'évaluation de reproductibilité et de la valeur d'évaluation mentale, et
dans lequel le générateur de résultats (99) délivre en outre en sortie un résultat de prédiction, sur la base du modèle de prédiction, de la valeur d'évaluation mentale correspondant à la valeur d'évaluation de reproductibilité indiquant un état dans lequel la reproductibilité est élevée.

7. Procédé d'évaluation d'état mental/physique mis en œuvre par ordinateur comprenant :
la détermination d'une valeur d'évaluation de reproductibilité quantifiant une reproductibilité élevée ou faible sur la base d'un résultat d'analyse concernant la reproductibilité d'un mouvement corporel prédéterminé d'un sujet ;
l'exécution d'une analyse pour quantifier la reproductibilité en utilisant un paramètre prédéterminé lié à la reproductibilité du mouvement corporel prédéterminé sur la base des informations indiquant l'état de mouvement, et la détermination de la valeur d'évaluation de reproductibilité sur la base du résultat d'analyse de celle-ci ;
la détermination d'une valeur d'évaluation mentale quantifiant un bon ou un mauvais état mental sur la base d'un résultat d'analyse concernant un état mental du sujet ;
l'exécution d'une analyse pour quantifier l'état mental en utilisant un paramètre prédéterminé lié à l'état mental sur la base des informations indiquant l'état mental, et la détermination de la valeur d'évaluation mentale sur la base du résultat d'analyse de celle-ci ;
le stockage de la valeur d'évaluation de reproductibilité et de la valeur d'évaluation mentale en association l'une avec l'autre ; et
la délivrance en sortie d'informations indiquant la pertinence entre le bon ou mauvais état mental sur la base de la valeur d'évaluation mentale et la reproductibilité élevée ou faible sur la base de la valeur d'évaluation de reproductibilité en affichant la valeur d'évaluation de reproductibilité et la valeur d'évaluation mentale ; et
l'acquisition d'informations indiquant l'état de mouvement du sujet effectuant de manière répétée le mouvement corporel prédéterminé en utilisant un acquéreur d'informations de mouvement (70) ;
dans lequel l'acquéreur d'informations de mouvement inclut un acquéreur d'images animées (32), un acquéreur de son (34) et un détecteur de mouvement (36) ; et
l'acquisition d'informations indiquant l'état mental du sujet en utilisant un acquéreur d'informations mentales (80) ;
dans lequel l'acquéreur d'informations mentales (80) inclut un acquéreur d'informations de perturbation (83) ;
dans lequel les informations de perturbation sont obtenues par la sélection et la saisie, par le sujet, d'un élément de stimulus externe, et par la détermination subjective, par le sujet, du degré de stimulus et la saisie, par le sujet, d'une valeur numérique dans l'acquéreur d'informations de perturbation.
